# EUROPÄISCHE PATENTANMELDUNG

(11) **EP 1 290 980 A1**
(43) Veröffentlichungstag der Anmeldung: **12.03.2003**
(21) Anmeldenummer: 01810879.5
(22) Anmeldetag: 11.09.2001
(51) Int. Cl.: A61B 17/04

(54) **Vorrichtung zum endoskopischen Nähen**

(71) Anmelder: Sulzer Orthopedics Ltd., 6340 Baar (CH)
(72) Erfinder: Largo, Mario, Dr. med., 8405 Winterthur (CH); Gross, Walter, 8475 Ossingen (CH)
(74) Vertreter: Manitz, Finsterwald & Partner Gbr

(57) **Zusammenfassung**

Die Erfindung betrifft eine endoskopische Nähhilfe mit einem Griffteil (1) und mit einem Schaftteil (2), an dessen Spitze (3) eine mit einem Faden (4) verbundene Nadel (5) befestigbar ist. Dadurch, dass die Nadel (5) eine Rundnadel (5a) mit annähernd konstanter Krümmung ist, dass die Spitze wie die Rundnadel gekrümmt ist und in Längsrichtung eine Nut (6) aufweist, in welche die Rundnadel (5a) auf ihrer ganzen Länge einschiebbar ist und dass im Schaftteil (2) ein vom Griffteil (1) aus betätigbarer Stössel (7) angebracht ist, über den die Rundnadel (5a) aus der Nut (6) geführt ausstossbar ist, wird ein schonendes und schnelles Nähen ermöglicht.

## Beschreibung

Die Erfindung handelt von einer endoskopischen Nähhilfe mit einem Griffteil und mit einem Schaftteil, an dessen Spitze eine mit einem Faden verbundene Nadel befestigbar ist.

Endoskopische und arthroskopische Eingriffe haben die Chirurgie der letzten Jahrzehnte enorm verändert. Durch das Setzen von wenigen "Ports" am menschlichen Körper wird der Zugang zu Regionen möglich, die früher nur über eine grössere Wunde in der Aussenhaut erreichbar waren. Diese neue Technik setzt wegen der beengten Platzverhältnisse und wegen der indirekten und beschränkten Sicht eine enorme Geschicklichkeit der Chirurgen voraus, die entsprechend einer langsamen Lernkurve erlernt werden muss. Jede Verbesserung im Instrumentarium, die die Operationszeit verkürzt und die die Lernkurve abkürzt, ist daher vorteilhaft.

Bei den meisten intrakorporalen Eingriffen müssen Nähte im Körper gemacht werden, ohne dass eine direkte Sicht möglich ist. So bietet die Firma Auto Suture Deutschland GmbH, Tempelsweg 26, D-47918 Tönisvorst unter dem Warenzeichen "ENDO STICH" ein Einweg-Naht-Instrument an, welches an seiner Spitze zu einer Zange ausgebildet ist, deren Backen gegenüberliegend die Spitzen einer dazwischen auf einem Abstand geklemmten Nadel aufnehmen können. Zwischen den gegenüberliegenden Spitzen der relativ kurzen Nadel - sie muss ja quer liegend durch den "Port" eingeführt werden - ist der Faden an der Nadel befestigt, der nach innen nachgezogen wird. Am Ort der Naht, werden die Backen auseinandergespreizt, wobei die Nadel in einer ersten Backe gehalten ist und über diese um den besagten Abstand und die für die zweite Backe vorgesehene Klemmlänge vorsteht. Die beiden zu nähenden Gewebeteile müssen nun durch Hilfsinstrumente zusammengeführt werden, um dicht daneben die Zange wie ein geöffnetes Maul über die beiden Gewebekanten zu schieben und zusammenzupressen. Dabei durchstösst die Nadel die beiden Gewebekanten, zieht den Faden nach und verankert sich in der gegenüberliegenden Backe. Anschliessend wird die Zange geöffnet und Nadel und Faden werden mit der die Verankerung bildenden Backe durch die Gewebekanten gezogen. Diese Anordnung ist in ihrer Anwendung beschränkt. Beide Gewebekanten müssen vorher zur Deckung gebracht und so herausgestülpt sein, dass die geöffnete Zange darüber eingefahren werden kann. Die beiden Gewebekanten dürfen zusammen nicht dicker als der Zwischenraum der zusammengepressten Backen sein, damit eine geordnete Übergabe der Nadel ohne zusätzliche Verletzung durch die Backen stattfindet. Ein genereller Nachteil besteht darin, dass beim Öffnen der Zange Nadel und Faden noch vollständig im Gewebe stecken und dass zunächst Ausziehkräfte in der Richtung der Nadelachse am Gewebe wirksam angebracht werden müssen, um die Nadel vollständig aus dem Gewebe zu ziehen, wobei die Zange in ihrer eigentlichen Ausziehrichtung, der Richtung ihrer Schaftachse, herausgezogen werden muss. Dadurch, dass diese Bewegungen in direkter Sicht kaum kontrollierbar sind, werden beim Ausziehen der geöffneten Zange durch die quer vorstehende Nadel ungewollt immer wieder Gewebeschäden verursacht, die zu einer störenden Narbenbildung führen.

Die Erfindung hat die Aufgabe, dem Chirurgen eine endoskopische Nähhilfe zu geben, die ein schonendes und schnelles Nähen erlaubt. Diese Aufgabe wird gemäss Anspruch 1 dadurch gelöst, dass die Nadel eine Rundnadel mit annähernd konstanter Krümmung ist, dass die Spitze wie die Rundnadel gekrümmt ist und in ihrer Längsrichtung eine Nut aufweist, in welche die Rundnadel auf ihrer ganzen Länge einschiebbar ist und dass im Schaftteil ein vom Griffteil aus betätigbarer Stössel angebracht ist, über den die Rundnadel aus der Nut geführt ausstossbar ist.

Ein Vorteil dieser Anordnung besteht darin, dass eine lange und gekrümmte Nadel verwendet werden kann, die mit der Spitze in Längsrichtung im "Port" eingebracht wird und daher weniger Querschnitt im Port benötigt. Dadurch, dass diese Nadel praktisch vollständig aus der Spitze ausstossbar ist, können auch dickere Gewebeteile durchstossen werden. Nadel und Faden verlassen die Spitze vollständig, sodass die Nähhilfe durch den Port zurückgezogen werden kann. Mit einem üblichen Nadelgreifer wird die aus der Gewebekante vorstehende Nadelspitze gefasst und soweit herausgezogen, dass der nachfolgende Faden gepackt werden kann, um daran die Nadel rückwärts durch den Port nach aussen zu ziehen. Es besteht kein Zwang die beiden Gewebekanten vor dem Nähen zusammenzufügen. Wenn beide Fadenenden mit einer Rundnadel versehen sind, können die beiden Gewebekanten unabhängig voneinander jeweils mit einer Rundnadel durchstossen werden und die eigentliche Annäherung der Gewebekanten erfolgt erst mit dem Setzen eines Knotens, wenn die Nähhilfe entfernt ist. Dabei können die Schlingen für den Knoten ausserhalb oder innerhalb des Körpers gebildet und zur Knotenbildung unter Zug mit einem "Pusher" nach innen verschoben werden. Die abhängigen Ansprüche 2 bis 13 bilden vorteilhafte Weiterbildungen der Erfindung.

Dadurch, dass die Spitze abnehmbar ist, kann sie als Wegwerfprodukt mit der darin eingebetteten Rundnadel vormontiert und steril verpackt werden. Sie muss nach dem Auspacken lediglich auf den Schaftteil aufgesteckt und dort beispielsweise mit einem Bajonettverschluss in axialer Richtung gesichert werden. Wegen der Krümmung von Spitze und Nut ist es sinnvoll, die Spitze als Spritzgussteil beispielsweise aus Kunststoff herzustellen, da durch die Formumkehr eine Bohrung mit daran anschliessender gekrümmter Nut möglich ist. Gleichzeitig entstehen bei grossen Stückzahlen der Spitze Stückkosten, die ein Wegwerfteil rechtfertigen.

Wenn man den Schaft zusätzlich in einem Bogen abwinkelbar und einstellbar macht, entsteht ein grosses Arbeitsfeld, das es ermöglicht auch bei entfernteren Gewebekanten eine Rundnadel mit einem Faden durchzuziehen und erst später diese Gewebekante mit dem Faden an ihre Gegenkante zu ziehen. Wenn der Faden an beiden Enden mit einer Rundnadel versehen ist, die jeweils in einer auswechselbaren Spitze für eine Nähhilfe eingesetzt ist, kann in beide Gewebekanten nacheinander zeitlich und örtlich getrennt der gleiche Faden eingezogen werden.

Wenn der Faden seitlich vor dem hinteren Ende der Rundnadel austritt, können grössere Kräfte beim Austreiben der Rundnadel durch den Stössel angebracht werden. Weitere Verbesserungen können im Anbringen eines Vibrators am Stössel bestehen, um beispielsweise härtere Gewebeschichten wie Knorpel nähen zu können. Mit einer Skala am Stössel lässt sich die Eindringtiefe der Rundnadel am Griffteil ablesen. Man kann eine solche Nähhilfe auch als Zwillingsteil mit zueinander entgegengesetzten Spitzen und mit zwei voneinander unabhängigen Stösseln ausführen, wenn Operationen ausgeführt werden, bei denen die zu nähenden Gewebekanten anfänglich weiter auseinander liegen.

Im folgenden wird die Erfindung anhand von Ausführungsbeispielen beschrieben. Es zeigen:
- Fig. 1: Schematisch die Anordnung einer Nähhilfe mit einer abnehmbaren Spitze und einen Faden mit zwei Rundnadeln, die jeweils in eine abnehmbare Spitze eingelegt sind;
- Fig. 2: schematisch einen stark vergrösserten Ausschnitt einer Spitze mit eingelegter Rundnadel;
- Fig. 3: schematisch einen stark vergrösserten Ausschnitt analog zu Fig. 2 mit einer anderen Fadenbefestigung und einem daran angepassten Stössel;
- Fig. 4: schematisch eine Anordnung analog zu Fig. 3 mit einem Stösselkopf, der sich an der Rundnadel zentriert;
- Fig. 5: schematisch einen Querschnitt von Fig.4;
- Fig. 6: schematisch eine weitere Anordnung analog zu Fig. 3 mit einem Stösselkopf mit einem Dorn, der in den Faden eindringt, ohne ihn ernsthaft zu verletzen; und
- Fig. 7: schematisch eine Anordnung wie in Fig. 6 mit einem zusätzlichen Gelenk, um die Spitze zusätzlich ausrichten zu können.

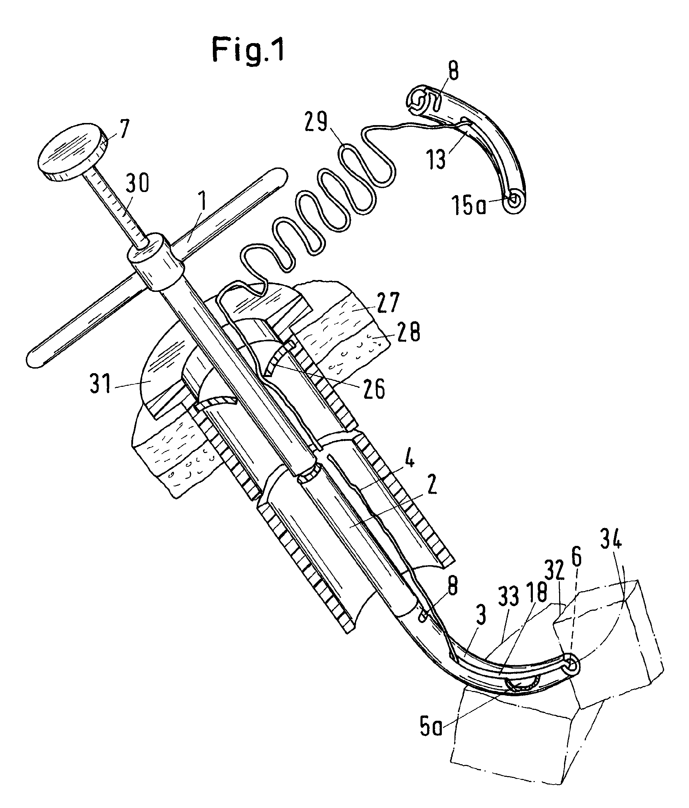

Die Darstellung in Fig. 1 zeigt eine Vorrichtung zum Nähen von organischen Geweben, die aus einem Griffteil 1, einem Schaftteil 2 und einer aufsetzbaren Spitze 3 besteht, in welche eine Rundnadel 5a mit einem Faden 4 eingelegt ist. Das zweite Fadenende endet in einer zweiten Rundnadel 15, die ihrerseits ebenfalls in einer Spitze 13 eingeschoben ist. Beide Spitzen 3, 13 können zeitlich nacheinander mit einem Bajonettverschluss 8 an einem Schaftteil 2 befestigt werden. Der Schaftteil 2 ist hohl und nimmt einen Stössel 7 auf, der aus dem Griffteil 1 herausragt. Der Stössel 7 steht um die Länge, die die Rundnadel 5a in der Spitze 3 einnimmt, nach oben vor. Der vorstehende Teil ist mit einer Skala 30 versehen, an der die Eindringtiefe des Stössels respektive der Nadel 5a, auf die der Stössel wirkt, abgelesen werden kann. Der an die Rundnadel 5a angrenzende Teil des Stössels 7 ist biegeelastisch, damit er der Rundnadel 5a in ihrer Nut 6 folgen kann. Die Spitze 3 besitzt einen Schlitz 18, in welchem der nach aussen vorstehende Faden 4 mitbewegt wird, wenn der Stössel 7 die Rundnadel 5a ausstösst.

In Fig. 1 ist weiterhin ein Port 31 mit einer innenliegenden Manchette 26 als Durchgang durch Aussenhaut 27 und Gewebe 28 dargestellt. Durch diese endoskopische Öffnung wurden vor dem Nähen chirurgische Eingriffe an Gewebekanten 32, 33 vorgenommen. Anschliessend wurde die Nähhilfe - Spitze 3 voraus - durch den Port 31 in die dargestellte Position zur Gewebekante 32 gebracht. Beim Betätigen des Stössels 7 wird nun die Rundnadel 5a entsprechend ihrer Krümmung auf einer Durchstosskurve 34 vollständig aus der Spitze 3 ausgestossen und ragt nun freigegeben mit ihrem Vorderteil aus der Gewebekante 32 heraus. In dieser Lage kann die Nadelspitze mit einem zweiten, greiferähnlichen Hilfsinstrument (nicht gezeigt) gepackt werden, während die Nähhilfe mit ihrem Schaft 2 und ihrer Spitze 3 durch den Port 31 herausgezogen wird. Mit dem Hilfsinstrument wird die Rundnadel 5a vollständig aus der Gewebekante 32 herausgezogen und durch den Port 31 entfernt, wobei der nachgezogene Faden 4. eine erste Schlaufe durch die Gewebekante 32 bildet. Die Rundnadel 5a ist nun mit ihrem Fadenende ausserhalb vom Port 31. Nachdem die erste, leere Spitze 3 von dem Schaft 2 gelöst ist, wird die zweite Spitze 13, in welcher die Rundnadel 15a eingeschoben ist, am Schaft 2 mit dem Bajonettverschluss 8 befestigt und durch den Port 31 an der zweiten Gewebekante 33 angesetzt, um ebenfalls die Rundnadel 15a durchzustossen und mit dem Hilfsinstrument die Nadelspitze zu fassen und durch den Port 31 nach aussen zu ziehen. Beide Rundnadeln 5a, 15a sind nun ausserhalb vom Port 31, während der Faden 4 durch die Gewebekanten 32, 33 hindurch eine grosse Schlaufe bildet.

Mit den Fadenenden an den Rundnadeln 5a, 15a werden beispielsweise ausserhalb vom Port Knoten vorbereitet, die mit einem Pusher-Werkzeug (nicht gezeigt) durch den Port 31 nach innen verschoben werden. Gleichzeitig werden die Gewebekanten 32, 33 durch die Verkleinerung der Schlaufe zusammengezogen. Nachdem die Knoten gesetzt sind, kann der überflüssige Faden abgeschnitten und herausgezogen werden.

Diese Operationstechnik hat den Vorteil, dass auch voneinander entfernte Gewebekanten 32, 33 vor dem Knoten mit mehreren Schlaufen verbunden werden können, um erst am Schluss die Schlaufen mit den Gewebekanten 32, 33 zusammenzuziehen und Knoten anzubringen.

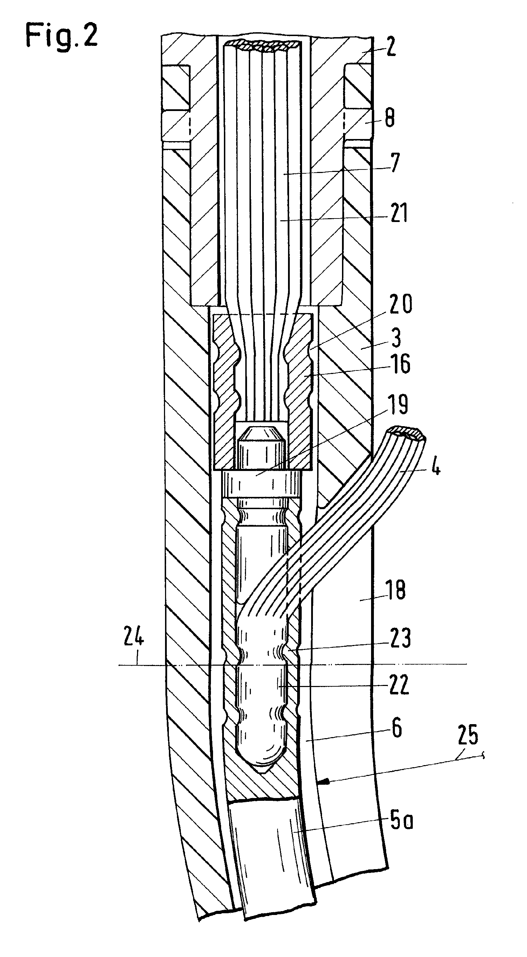

In den restlichen Fig. 2 bis 6 sind verschiedene Konstruktionen für eine abnehmbare Spitze 3 und für die Gestaltung von Stössel 7 und Rundnadel 5a an ihrer Trennstelle aufgezeigt. Es sind gleiche Hinweiszeichen für gleiche Fuktionen verwendet.

In Fig. 2 ist die abnehmbare Spitze 3 mit einem Bajonettverschluss 8 am Schaft 2 befestigt. Der Stössel 7 ist mit seinem unteren Teil, der in die Nut 6 der Spitze einschiebbar ist, als ein Paket aus mehreren Federdrähten 21 ausgeführt, die in einem Kopf 16 befestigt sind. Die Befestigung erfolgt durch Einrollen am Kopf 16, wobei Rillen 20 entstehen. Die parallel zueinander liegenden Federdrähte lassen einerseits eine Biegung des Stössels 7 in der Spitze 3 zu und füllen andererseits den Schaft so aus, dass ein Knicken der einzelnen Federdrähte 21 nicht möglich ist. Die Rundnadel 5a endet mit einem eingerollten Stopfen 19, an dem sich der Stössel 7 mit seinem Kopf 16 während des Ausstossens zentriert. Der Faden 4 ist durch plastische Deformation am Ende der Rundnadel 5a als Pfropfen 22 verpresst und tritt seitlich zum Stopfen 19 aus der Rundnadel aus. In einer Ebene 24, die senkrecht zum Schaft 2 steht, beginnt die Krümmung der Rundnadel 5a und der Nut 6. Die Krümmung der Spitze 3 liegt in der Zeichenebene. Wegen der gekrümmten gemeinsamen Führung ist die Lage der Rundnadel vorgegeben, sodass der Faden 4 zwangsläufig eine richtige Position im Schlitz 18 der Spitze einnimmt.

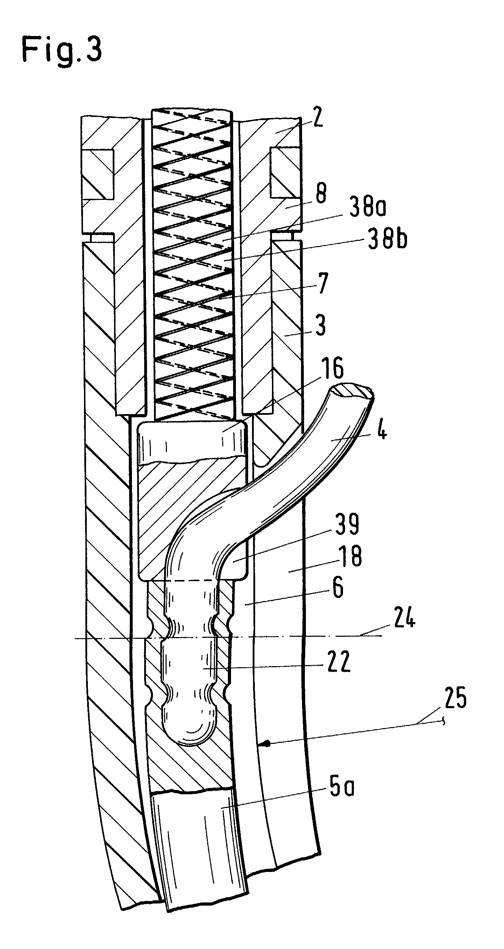

Eine weitere Ausführung ist mit Fig. 3 gezeigt, in der die Spitze 3 ebenfalls mit einem Bajonettverschluss 8 abnehmbar befestigt ist. Der Stössel 7 ist in seinem unteren Teil aus zwei ineinander geschraubten Schraubenfedern 38a, 38b zusammengesetzt, die sich in der Richtung der Längsachse des Schaftes 2 gegenseitig berühren und daher nicht komprimierbar sind, aber trotzdem in der Lage sind, einer Biegung entsprechend einer Krümmung 25 in der Nut 6 zu folgen. Der Kopf 16 des Stössels 7 besitzt eine seitliche Ausnehmung 39, mit welcher sich der Stössel 7 am Faden 4 zur Rundnadel 5a zentriert und gleichzeitig den Faden 4 zur Nut 18 seitlich freigibt.

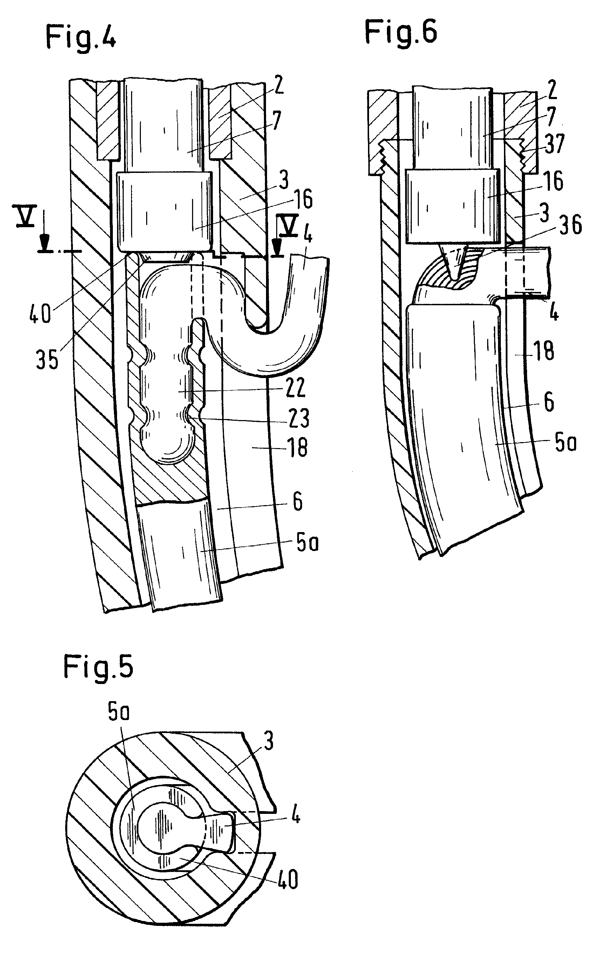

In einer Ausführung gemäss Fig. 4 und 5 wird auf einen Stopfen 19 aus Fig. 2 verzichtet und die Hohlnadel 5a ist an ihrem Ende seitlich ausgenommen, um den Faden 4 in Richtung der Nut 18 freizugeben. Der Stösselkopf 16 zentriert sich mit einer konischen Zentrierung 35 an einem axial vorstehenden Rand 40 des Endes der Rundnadel. Gemäss Fig. 5 umgibt der Rand 40 den Faden auf einem Winkel von mehr als 180°, um so für den Kopf 16 eine Zentrierung in jeder Richtung quer zur Längsachse zu bilden.

Mit Fig. 6 ist eine Ausführung gezeigt, bei der Schaft 2 und Spitze 3 über ein Gewinde 37 verschraubbar sind. Der Kopf 16 vom Stössel 7 endet mit einem Dorn 36, welcher so stumpf ist, dass er Fibrillen des Fadens 4 nicht zerstört, wohl aber den Faden seitlich wegdrückt und sich zur Rundnadel 5a zentriert.

Eine solche Lösung ist kostengünstig bezüglich Herstellung, sie erlaubt die Verwendung von handelsüblichen Rundnadeln und sie beansprucht wenig Raum.

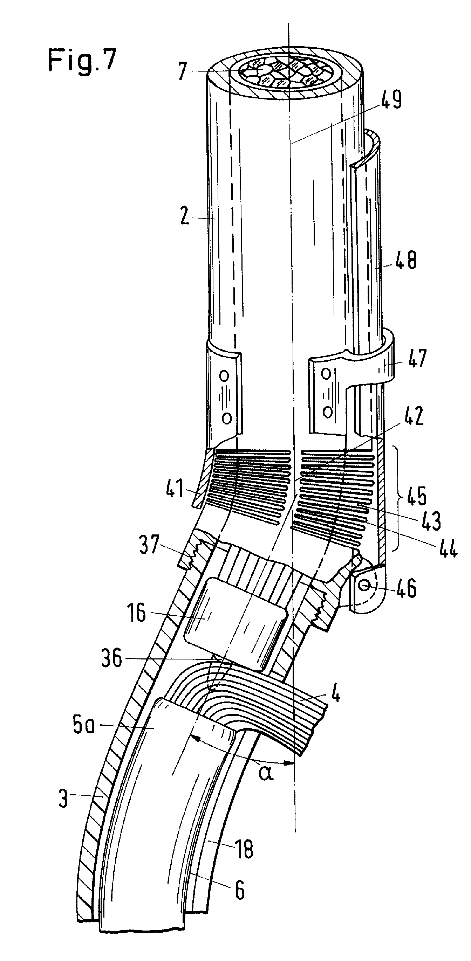

Mit Fig. 7 ist eine weitere Verbesserung der Anordnung von Fig. 6 gezeigt. Der Schaftteil 2 besitzt oberhalb vom Gewinde 37 ein Biegegelenk 45, welches über eine Zug- und Schubstange 48 vom Griffteil her betätigbar ist. Damit kann die gekrümmte Spitze 3 soweit um einen Winkel α zurückgestellt werden, dass die Längsachse 49 vom Schaftteil eine Sekante zur gekrümmten Spitze 3 bildet, um mit möglichst wenig Ausladung in einen Port einzufahren. Andererseits kann durch die Zug- und Schubstange 48 die Spitze 3 in der entgegengesetzten Richtung soweit geschwenkt werden, dass die Rundnadel 5a in der Richtung der Längsachse 49 aus einer Gewebekante nach oben austritt. Ein oder mehrere Haltebügel 47 führen dabei die Zug- und Schubstange 48, die über einen Bolzen 46 an einem Auge unterhalb des Biegegelenks 45 befestigt ist. Die Auslenkung um den Winkel α wird durch einen seitlichen Anschlag 41 begrenzt, während bei Zugbelastung der Haltebügel 47 mit seinem Spiel zur Zug- und Schubstange eine Auslenkung begrenzt. Eine weitere Begrenzung der Schwenkbewegung findet im Biegegelenk 45 statt, welches aus zwei Biegefedern 42 und vielen Ringteilen 44 besteht. Feine Schlitze 43 sind beispielsweise durch funkenerosives Drahtschneiden (spark erosion wire cutting) von zwei Seiten herausgearbeitet worden, wobei die Biegefedern 42 und die die Biegung begrenzenden Ringteile 44 stehen geblieben sind.

Unabhängig von den gezeigten Ausführungsbeispielen lässt sich eine solche Nähhilfe auch ohne Port mit Vorteil an freiliegenden Gewebekanten 32, 33 verwenden, da auch bei grösseren Eingriffen solche Gewebekanten tiefer im Körper liegen und die gezeigte Nähhilfe im Gegensatz zu einer Zange, die eine Rundnadel packt und eine notwendige Drehung mitmacht, weniger Platz beansprucht.

## Patentansprüche

1. Endoskopische Nähhilfe mit einem Griffteil (1) und mit einem Schaftteil (2), an dessen Spitze (3) eine mit einem Faden (4) verbundene Nadel (5) befestigbar ist, **dadurch gekennzeichnet, dass** die Nadel (5) eine Rundnadel (5a) mit annähernd konstanter Krümmung ist, dass die Spitze wie die Rundnadel gekrümmt ist und in Längsrichtung eine Nut (6) aufweist, in welche die Rundnadel (5a) auf ihrer ganzen Länge einschiebbar ist und dass im Schaftteil (2) ein vom Griffteil (1) aus betätigbarer Stössel (7) angebracht ist, über den die Rundnadel (5a) aus der Nut (6) geführt ausstossbar ist.

2. Nähhilfe nach Anspruch 1, **dadurch gekennzeichnet, dass** die Spitze (3) abnehmbar oder als Wegwerfprodukt mit dem Schaftteil (2) verbindbar ist.

3. Nähhilfe nach Anspruch 2, **dadurch gekennzeichnet, dass** die Verbindung ein Bajonettverschluss (8) ist.

4. Nähhilfe nach einem der vorangehenden Ansprüche, **dadurch gekennzeichnet, dass** die Spitze (3) ein Spritzgussteil beispielsweise aus Kunststoff ist.

5. Nähhilfe nach einem der vorangehenden Ansprüche, **dadurch gekennzeichnet, dass** der Schaftteil (2) zur Spitze hin in einem zusätzlichen Bogen (9) abgewinkelt ist.

6. Nähhilfe nach Anspruch 5, **dadurch gekennzeichnet, dass** eine Abwinklung (10) des Schaftteils (2) in verschiedenen Winkelstellungen α einstellbar ist.

7. Nähhilfe nach Anspruch 5 oder 6, **dadurch gekennzeichnet, dass** die Abwinklung (10) des Schaftteils (2) in der gleichen Ebene wie die Krümmung der Spitze liegt.

8. Nähhilfe nach einem der vorangehenden Ansprüche, **dadurch gekennzeichnet, dass** am Griffteil ein Vibrator (11) aufsetzbar ist, um die Rundnadel (5a) über den Stössel (7) mit gezielten kleinen Schlägen aus der Spitze (3) zu treiben.

9. Nähhilfe nach einem der vorangehenden Ansprüche mit einem Faden (4) dessen beide Enden jeweils mit einer Rundnadel (5a) versehen sind.

10. Nähhilfe nach Anspruch 9, **dadurch gekennzeichnet, dass** beide Rundnadeln (5a, 5b) jeweils in einer auswechselbaren Spitze angelegt sind.

11. Nähhilfe nach einem der vorangehenden Ansprüche mit einer Rundnadel (5a), bei der der Faden (4) vor dem der Nadelspitze gegenüberliegenden Ende (12) seitlich austritt, um grössere Ausstosskräfte auf das Ende (12) der Nadel (5a) zu bringen.

12. Nähhilfe nach einem der vorangehenden Ansprüche, **dadurch gekennzeichnet, dass** die Eintauchlänge vom Stössel (7) am Griffteil an einer Skala (14) ablesbar ist.
